# DEMANDE DE BREVET EUROPEEN

(11) **EP 0 847 741 A1**
(43) Date de publication de la demande: **17.06.1998**
(21) Numéro de dépôt: 96402711.4
(22) Date de dépôt: 12.12.1996
(51) Int. Cl.: A61G 9/00, A61J 19/00, A61F 5/44

(54) **Poche souple à manchon d'ouverture**

(71) Demandeur: Cailleteau, Benoît, 13008 Marseille (FR)
(72) Inventeur: Cailleteau, Benoît, 13008 Marseille (FR)
(74) Mandataire: Hasenrader, Hubert

(57) **Abrégé**

Poche présentant une enveloppe externe (12) souple comprenant un col (16) ouvert (17) suivi par une partie évasée (18). La poche comporte un élément de renfort (22) constitué par un manchon relativement rigide et flexible s'étendant dans le col à partir d'une première extrémité (22a) située au voisinage de l'ouverture (17) du col, jusqu'à une deuxième extrémité (22b) située du côté de la partie évasée (18). Le manchon présente, au voisinage de la base du col, une découpe (24) de part et d'autre de laquelle sont délimitées une première et une deuxième portion (23a, 23b). La deuxième portion (23b) est munie de deux pattes (26) sensiblement transversales qui se font face et qui ont des extrémités latérales libres (26a). Dans la configuration aplatie de la poche dans laquelle l'ouverture est fermée, le manchon est lui-même aplati et les deux pattes se trouvent l'une contre l'autre, tandis que, pour amener le col (16) dans sa configuration sensiblement tubulaire et ouvrir l'ouverture (17), le manchon (22) est susceptible d'être déformé élastiquement de telle sorte que la première portion (23a) adopte une configuration sensiblement tubulaire, cependant que les extrémités latérales (26a) des pattes (26) sont écartées l'une de l'autre.

## Description

La présente invention concerne une poche présentant une enveloppe externe souple comprenant un col suivi par une partie évasée, le col ayant une extrémité libre délimitant une ouverture, la poche étant susceptible d'adopter une configuration aplatie dans laquelle l'ouverture est refermée, cependant que le col est susceptible d'adopter une configuration sensiblement tubulaire dans laquelle l'ouverture est ouverte, la poche étant, en outre, munie d'un élément de renfort disposé au moins au voisinage de l'extrémité libre du col.

Des poches de ce type sont utilisées pour recevoir des déchets sous forme généralement liquide, notamment des déchets d'origine humaine tels que de l'urine ou des vomissures. Ces poches se présentent normalement dans leur forme aplatie et sont ouvertes pour recevoir les déchets. A cette occasion, la poche est maintenue au voisinage de l'extrémité libre du col pour contraindre ce dernier à adopter sa configuration sensiblement tubulaire. Une fois que les déchets ont été introduits, il suffit de relâcher l'extrémité libre du col pour que celui-ci reprenne naturellement sa configuration aplatie, refermant ainsi l'ouverture.

De telles poches sont couramment réalisées au moyen de feuilles minces de papier ou de matière plastique, éventuellement transparentes, dotées de la souplesse voulue. Pour l'ouverture, l'élément de renfort est déformé par l'utilisateur, ce qui contraint la partie du col disposée autour de cet élément à adopter la configuration tubulaire souhaitée.

Les dimensions transversales de l'ouverture sont choisies de manière appropriée à l'usage que l'on souhaite faire de la poche. Généralement, surtout lorsque la poche est destinée à recevoir des urines ou des vomissures, l'ouverture doit pouvoir être manipulée avec une seule main, de sorte que ses dimensions transversales sont nettement inférieures aux dimensions transversales courantes de la poche. Par exemple, lorsque la poche est utilisée comme urinal, sa largeur maximale et celle de l'ouverture peuvent respectivement varier entre 15 et 20 cm et entre 4 et 10 cm, selon que l'utilisateur est un adulte ou un enfant.

La présence de l'élément de renfort permet d'ouvrir l'ouverture de l'extrémité supérieure du col en rapprochant les deux bords longitudinaux de cet élément qui déterminent sa largeur maximale dans la configuration aplatie. Toutefois, du fait de la souplesse du matériau qui constitue l'enveloppe externe de la poche, il existe un risque que seul le col soit convenablement ouvert, tandis que le reste de l'enveloppe externe reste plat, les feuilles qui constituent cette enveloppe ayant naturellement tendance à rester "collées" les unes aux autres. Par conséquent, lors de l'utilisation, il existe un risque de reflux vers l'extérieur pour le produit versé dans la poche.

L'invention a pour but de remédier à cet inconvénient en proposant une poche pour laquelle le passage à la configuration tubulaire du col assure l'écartement des parois de l'enveloppe externe non seulement dans la région de ce col, mais également au moins dans la région de la partie évasée proche du col.

Ce but est atteint grâce au fait que l'élément de renfort est constitué par un manchon relativement rigide et flexible disposé à l'intérieur de la poche, ce manchon s'étendant dans le col à partir d'une première extrémité située au voisinage de l'ouverture, jusqu'à une deuxième extrémité située du côté de la partie évasée, que le manchon présente, au voisinage de la base du col raccordée à la partie évasée, une découpe de part et d'autre de laquelle sont délimitées une première portion située du comté de la première extrémité du manchon et une deuxième portion située du côté de la deuxième extrémité dudit manchon, la deuxième portion étant munie d'au moins deux pattes sensiblement transversales qui se font face et qui ont des extrémités latérales libres, et que, dans la configuration aplatie de la poche, le manchon est lui-même aplati et les deux pattes se trouvent l'une contre l'autre, tandis que, pour amener le col dans sa configuration sensiblement tubulaire et ouvrir l'ouverture, le manchon est susceptible d'être déformé élastiquement de telle sorte que la première portion adopte une configuration sensiblement tubulaire, cependant que les extrémités latérales des pattes sont écartées l'une de l'autre.

On comprend que, grâce à cette conformation, la déformation du manchon permet non seulement d'amener le col à son diamètre d'ouverture grâce à la première portion du manchon, mais également d'écarter les parois de la poche au moins dans la partie évasée de son enveloppe externe du fait de l'écartement des pattes qui équipent la deuxième portion du manchon.

La précision selon laquelle le manchon est "relativement rigide et flexible" signifie qu'il est réalisé dans un matériau doté d'une rigidité supérieure à celle de la paroi externe de la poche. Par exemple, le manchon peut être réalisé dans un carton léger ou dans un plastique d'épaisseur supérieure à celle du plastique qui constitue la paroi externe.

Selon un mode de réalisation particulièrement avantageux, le manchon comporte deux plaquettes analogues qui sont fixées à l'intérieur de la poche, respectivement sur ses deux faces internes en regard. Chaque plaquette présente une encoche sensiblement transversale de part et d'autre de laquelle s'étendent une première et une deuxième portion correspondant respectivement à la première et à la deuxième portion du manchon. Les deux pattes sont constituées par les portions des plaquettes situées entre leur découpe respective et celle de leurs extrémités qui est dirigée vers l'intérieur de la poche. Chaque plaquette présente deux bords longitudinaux qui sont respectivement situés au voisinage des deux bords longitudinaux que présente le col lorsque la poche est dans sa configuration aplatie.

Grâce à cette conformation, les plaquettes étant naturellement planes, elles permettent naturellement l'obtention de la configuration aplatie de la poche. Pour ouvrir cette dernière, l'utilisateur manipule le col dans la région de ses deux bords longitudinaux, ce qui a pour effet d'écarter les régions centrales des plaquettes et de rapprocher leurs bords longitudinaux jusqu'à obtenir, pour la première portion du manchon, la configuration sensiblement tubulaire. Du fait que les extrémités latérales des pattes sont libres, celles-ci conservent naturellement leur configuration plane ou sensiblement plane, de sorte qu'elles s'écartent l'une de l'autre même dans la région de leurs extrémités latérales, ce qui a pour effet d'écarter les parois en regard de la poche.

L'invention sera bien comprise et ses avantages apparaîtront mieux à la lecture de la description détaillée qui suit, d'un mode de réalisation représenté à titre d'exemple non limitatif. La description se réfère aux dessins annexés sur lesquels :
- la figure 1 est une vue en élévation d'une poche conforme à l'invention, dans sa configuration aplatie,
- la figure 2 est une vue en perpective selon la flèche II de la figure 1, montrant l'extrémité supérieure de la poche dans sa configuration ouverte, et
- la figure 3 est une vue partielle en élévation de l'extrémité supérieure de la poche, dans la configuration aplatie, selon une variante de réalisation du manchon.

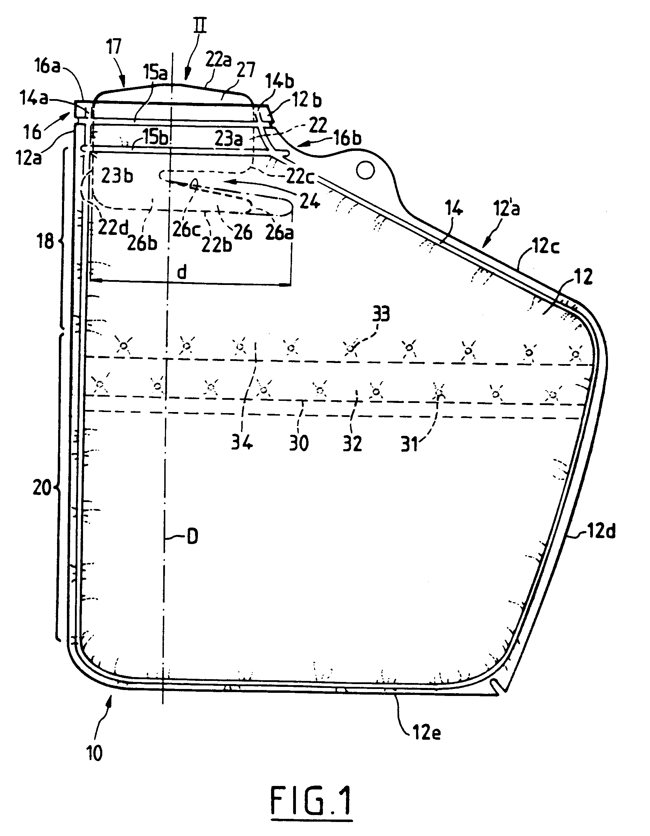

La figure 1 montre une poche de sécurité 10, par exemple destinée à être utilisée comme urinal. Cette poche comporte une enveloppe externe 12 qui est souple. Cette enveloppe est par exemple réalisée à partir de deux feuilles minces de matière plastique découpées de manière convenable et soudées l'une sur l'autre sur pratiquement l'intégralité du contour, par une ligne de soudure 14.

L'enveloppe 12 comprend un col 16 qui, dans le sens allant vers le fond de la poche, est suivi par une partie évasée 18, elle-même suivie par une partie utile 20 de la poche qui présente une largeur sensiblement constante.

Plus précisément, dans l'exemple représenté, un bord 12a de l'enveloppe 12 est sensiblement rectiligne à partir de l'extrémité libre du col 16, tandis que le bord opposé 12'a présente tout d'abord une première portion 12b qui est située dans la région du col 16 et qui est sensiblement parallèle au bord 12a dans cette région, ou qui a tout au plus tendance à s'écarter légèrement de ce bord 12a. Le bord 12'a présente ensuite une deuxième portion 12c qui va en s'écartant du bord 12a. C'est cette portion 12c qui définit la portion évasée 18 de l'enveloppe externe. Enfin, la partie utile 20 de la poche est délimitée entre le bord 12a et la portion 12d du bord 12'a qui lui fait face. Cette portion 12d peut être sensiblement parallèle au bord 12a ou, comme dans l'exemple représenté, avoir légèrement tendance à se rapprocher de ce dernier. D'autres configurations sont encore possibles, l'essentiel étant d'obtenir un volume interne correspondant à l'utilisation que l'on souhaite faire de la poche. Enfin, le bord 12a et la portion 12d du bord 12'a sont raccordés par le fond 12e de la poche, qui est situé à l'opposé de l'extrémité libre 16a du col 16.

On a indiqué précédemment que la soudure 14 était pratiquement continue. En fait, comme on le voit sur la figure 1, cette soudure forme une ligne continue entre deux extrémités 14a et 14b entre lesquelles elle est interrompue. Ces extrémités sont situées de chaque comté du col 16. Ainsi, l'extrémité 16a du col présente une ouverture 17 qui s'étend sur sensiblement toute sa largeur. Dans toute la suite, on considérera que la longueur des différents éléments de la poche est définie dans la direction, donnée par l'axe D, qui va de l'extrémité libre 16a du col au fond 12e de la poche, tandis que la largeur des différents éléments est définie transversalement à cette longueur.

La poche comporte un élément de renfort 22 constitué par un manchon relativement rigide et flexible qui est disposé à l'intérieur de la poche, dans la région du col 16. Plus précisément, ce manchon s'étend dans le col à partir d'une première extrémité 22a disposée au voisinage de l'ouverture 17, jusqu'à une deuxième extrémité 22b qui est située à l'intérieur de la poche, dans la région de la partie évasée 18. Au voisinage de la base 16b du col 16 (c'est-à-dire au voisinage de la zone de raccordement entre le col et la partie évasée), ce manchon présente une découpe 24 de part et d'autre de laquelle sont délimitées une première portion 23a et une deuxième portion 23b. La portion 23a s'étend entre la découpe 24 et l'extrémité 22a du manchon, tandis que la portion 23b s'étend entre la découpe et l'extrémité 22b.

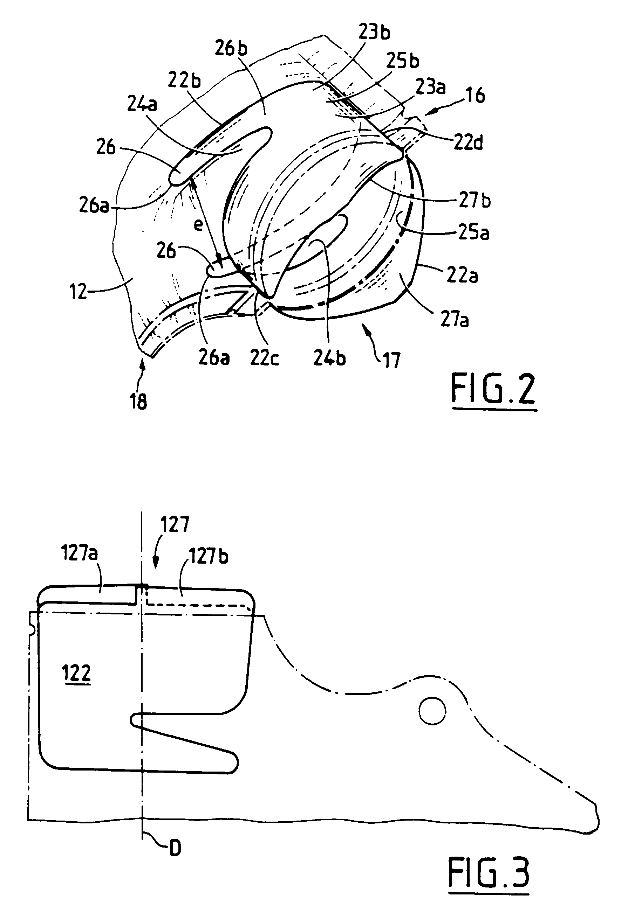

La deuxième portion 23b est munie de deux pattes 26 sensiblement transversales à l'axe D. Dans la position aplatie de la poche, les deux pattes 26 sont disposées l'une contre l'autre, ce qui explique que l'on n'en voie qu'une seule sur la figure 1. En revanche, sur la figure 2, les deux pattes 26 sont visibles. Les extrémités latérales 26a des pattes sont libres, c'est-à-dire que ces extrémités ne se rejoignent pas et ne sont pas fixées entre elles. Ainsi, la découpe 24 comporte deux encoches 24a et 24b qui sont sensiblement transversales, et, éventuellement, une ligne de découpe séparant les extrémités 26a des deux pattes.

Le manchon est fixé à l'intérieur de la poche et, comme on le voit sur la figure 1, il est fixé dans la région du col à l'aide de deux soudures transversales 15a et 15b.

La première portion 23a du manchon s'étend sur sensiblement toute la largeur du col 16, c'est-à-dire que la distance qui, dans la configuration aplatie, sépare les bords longitudinaux 22c et 22d du manchon dans sa première portion est sensiblement égale à la largeur du col donnée par la distance entre les extrémités 14a et 14b de la soudure 14. La deuxième portion s'étend globalement dans le prolongement de la première, vers le fond 12e de la poche. Toutefois, comme on l'a indiqué en traits mixtes interrompus sur la figure 1, la dimension transversale d maximale de la deuxième portion dans la région des pattes peut être supérieure à la largeur courante de la première portion. cette disposition vise à augmenter encore l'effet des pattes qui est d'écarter les deux parois de la poche dans la région située en dessous du col.

On voit en effet sur la figure 2 la partie supérieure de la poche dans la configuration ouverte de cette dernière. Pour parvenir à cette configuration, le manchon est déformé élastiquement, de telle sorte que sa première portion 23a adopte une configuration sensiblement tubulaire. En revanche, grâce à la découpe 24 et au fait que les extrémités latérales des pattes sont libres, ces dernières restent sensiblement rectilignes. Elles sont raccordées à la partie courante du manchon par leurs extrémités internes 26b opposées aux extrémités 26a. Lorsque l'on déforme le manchon comme sur la figure 2, les extrémités internes 26b prennent un écartement donné, fonction du diamètre de la première portion du manchon. Les pattes 26 restent alors sensiblement rectilignes, de telle sorte que pratiquement sur toute leur longueur elles conservent un écartement e sensiblement égal à l'écartement entre leurs extrémités internes respectives.

Ainsi, lorsque l'on déforme le manchon, le col 16 de la poche qui est disposé autour de la première portion 23a du manchon adopte une configuration tubulaire dont le diamètre est déterminé par l'écartement maximal des parois en regard du manchon. Les pattes 26 s'étendent dans la partie évasée 18 de la poche et tendent à écarter cette dernière qui, dans un plan perpendiculaire à celui de la figure 1 passant par l'axe D, adopte sensiblement le diamètre de la première portion 23a du manchon et qui, dans la région des extrémités des pattes 26 (région qui est éloignée de l'axe D) adopte l'écartement e. Ainsi, les parois de la poche se décollent l'une de l'autre dans la région de la partie évasée.

Dans l'exemple représenté, la partie évasée 18 est obtenue en faisant en sorte que la portion 12c du bord 12'a s'écarte du bord 12a. Dans ces conditions, il est avantageux que les pattes soient dirigées vers cette portion 12c.

Si la portion évasée avait une autre conformation, par exemple une forme tronconique symétrique par rapport à l'axe D, on pourrait choisir de réaliser un manchon analogue à celui de la figure 1 en dirigeant ses pattes vers l'un ou vers l'autre des deux bords de la portion tronconique. On pourrait également choisir d'utiliser un manchon comportant quatre pattes analogues aux pattes 26, respectivement disposées de part et d'autre de l'axe D de manière à bien écarter les parois de la poche dans la partie évasée, dans deux directions opposées partant transversalement à l'axe D.

Globalement, la première portion 23a du manchon présente une symétrie par rapport à l'axe D. Notamment, dans la configuration ouverte, cet axe correspond à l'axe géométrique du tube que forme la première portion 23a du manchon. Dans ces conditions, le diamètre de ce tube est donné par l'écartement des deux parois opposées du manchon, mesurées selon un plan passant par l'axe D et perpendiculaire au plan de la poche dans sa configuration aplatie. Il est avantageux de faire en sorte que l'écartement e des extrémités des pattes soit sensiblement égal à ce diamètre. Pour ce faire, comme on le voit sur la figure 1, la découpe qui ménage les deux pattes s'étend sensiblement jusqu'à l'axe D à partir d'un bord longitudinal 22c que présente le manchon dans sa configuration aplatie. Dans l'exemple représenté, ce bord 22c est celui des bords longitudinaux du manchon qui est voisin de la portion 12c du bord de la poche.

De préférence, il est même souhaitable que cette découpe s'étende, dans la direction allant du bord 22c au bord opposé 22d, au-delà de l'axe D. Ainsi, les extrémités 26b des pattes sont reliées à la partie courante du manchon dans une région sur laquelle les parois du manchon divergent, c'est-à-dire qu'elles vont en s'écartant l'une de l'autre, lorsque l'on ouvre le manchon. Les pattes étant relativement rigides, elles ont ainsi tendance, lorsque le manchon est ouvert, à s'écarter en suivant la direction divergente donnée par les parois du manchon dans la région de leurs extrémités internes 26b. Ainsi, même si les parois de la poche ont quelque peu tendance à résister à l'écartement des pattes 26, on peut s'assurer que l'espacement e entre les extrémités 26a de ces pattes soit suffisant. Pour que les pattes aient la rigidité souhaitée, il est toutefois préférable que la découpe 24 ne s'étende pas trop au-delà de l'axe D mais dépasse seulement de quelques millimètres.

Selon l'exemple particulièrement avantageux représenté, le manchon comporte deux plaquettes analogues 25a et 25b. Ces plaquettes sont naturellement planes et sont réalisées en carton léger ou en plastique relativement rigide et flexible. Les plaquettes sont respectivement collées sur les faces internes de la poche qui se trouvent en regard l'une de l'autre lorsqu'elle occupe sa configuration aplatie. Les plaquettes sont fixées de telle sorte que leurs extrémités longitudinales soient adjacentes deux à deux et respectivement voisines des deux bords longitudinaux que présentent le col 16 dans la configuration aplatie de la poche. Ainsi, les deux bords longitudinaux des plaquettes définissent respectivement les bords 22c et 22d du manchon précédemment évoqué et ne sont pas désignés par d'autres références.

Pour former la découpe 24, chaque plaquette présente une encoche sensiblement transversale, respectivement 24a et 24b. Pour chaque plaquette, une première et une deuxième portion qui s'étendent respectivement de part et d'autre de cette encoche et qui correspondent respectivement aux première et aux deuxième portions du manchon. Il faut noter qu'une autre découpe rendant les extrémités latérales des pattes libres n'est pas nécessaire dans la mesure où ces extrémités coïncident normalement avec les bords longitudinaux des plaquettes qui sont initialement séparés l'un de l'autre et sont simplement maintenus en position grâce au fait que la largeur des premières portions des plaquettes est sensiblement égale à celle du col.

On pourrait également réaliser le manchon en une seule pièce en le dotant, dans la région des bords 22c et 22d, de moyens formant charnière. Si tel était le cas, une charnière pourrait s'étendre sur toute la longueur du bord 22d mais, pour le bord 22c, elle ne s'étendrait que sur la longueur de la première portion 23a du manchon.

Comme on le voit sur les dessins, les extrémités latérales 26a des pattes sont arrondies pour éviter de blesser les parois de la poche lors de leur écartement.

On remarque également que les bords supérieurs 26c des pattes, c'est-à-dire les bords qui sont tournés vers l'ouverture de la poche, ne sont pas tout à fait transversaux mais sont légèrement inclinés de manière à s'étendre sensiblement parallèlement à la portion évasée de l'enveloppe externe de la poche, plus précisément à son bord 12c. De ce fait, les pattes ont une forme tout à fait adaptée à celle de la portion évasée de la poche dans laquelle elles se trouvent et ouvrent cette dernière sans difficulté.

La découpe 24 est située au voisinage de la base du col 16 et, de préférence, légèrement en dessous de cette base dans le sens allant vers le fond 12e de la poche.

On voit sur les figures que le manchon 22 comporte un prolongement 27 qui s'étend vers l'extérieur au-delà de l'ouverture 17. Ce prolongement est muni de moyens pour faciliter le passage de la configuration aplatie du manchon à sa configuration tubulaire. Il s'agit de moyens de préhension qui permettent de bien saisir les parois en regard du manchon pour les écarter légèrement tout en rapprochant les bords longitudinaux 22c et 22d. Dans l'exemple des figures 1 et 2, ces moyens de préhension sont simplement constitués par deux languettes 27a et 27b qui équipent respectivement les deux parois du manchon parallèles aux deux parois en regard de la poche.

La figure 3 montre une variante de réalisation, sur laquelle, pour simplifier, le contour de l'enveloppe est simplement indiqué par des traits mixtes interrompus. On voit que le prolongement 127 du manchon 122 présente deux languettes, respectivement 127a et 127b, qui, contrairement à l'exemple de la figure 2, n'ont pas la même forme. En effet, ces deux languettes présentent deux surfaces de préhension décalées. Pour réaliser ces surfaces, il suffit de découper le prolongement 127 de chacune des deux plaquettes du manchon ou de chacune de ses deux parois en regard, de manière asymétrique par rapport à l'axe D. Ainsi, une portion découpée du prolongement de la première plaquette se trouve en regard d'une portion restée pleine du prolongement de la deuxième plaquette.

Lorsque la poche est destinée à être utilisée comme urinal, la partie supérieure 27 ou 127 du manchon 22 ou 122 peut être équipée d'une pièce d'adaptation au sexe (masculin ou féminin) de l'utilisateur, en une seule pièce avec le manchon ou sous la forme d'une pièce additionnelle.

Comme on le voit sur la figure 1, la poche peut comporter un clapet de sécurité, par exemple constitué par plusieurs feuilles internes disposées contre chacune des parois de la poche. Dans l'exemple représenté, ce clapet comporte ainsi, pour chaque paroi de la poche, une première paire de feuilles 30 qui s'étend à partir de l'ouverture jusqu'à une région médiane de la poche. Le clapet comporte également une deuxième paire de feuilles 32 qui s'étend à l'intérieur de la paire 30, également à partir de l'ouverture, sur une longueur légèrement moindre que celle de la paire 30. La paire de feuilles 30 et la paire de feuilles 32 sont soudées ensemble par des points de soudure 31 régulièrement espacés. Le clapet comporte enfin une troisième paire de feuilles 34 qui s'étend à l'intérieur de la paire 32 sur une longueur encore moindre que celle de la paire 32 et qui est soudée aux paires 30 et 32 par des points de soudure 33 qui sont régulièrement espacés et décalés par rapport au point de soudure 31.

Dans ce cas, la "paroi interne" de la poche est définie, dans la région du col, par les faces internes en regard des deux feuilles de la paire 34 la plus interne. Le manchon est donc au contact de ces faces internes. Ainsi, les premières (respectivement les deuxièmes) feuilles de chaque paire 30, 32, 34 sont prises en "sandwich" entre une première (respectivement une deuxième) paroi du manchon (plaquette 25a ou 25b) et une première (respectivement une deuxième) paroi de l'enveloppe externe 12. Les soudures 15a et 15b soudent donc ensemble, de chaque côté de la poche, une paroi du manchon, une feuille de chaque paire 30, 32, 34 et une paroi de l'enveloppe 12.

Bien entendu, la poche munie du manchon selon l'invention peut comporter un clapet de sécurité différent ou, selon les applications souhaitées, ne pas comporter de clapet.

## Revendications

1. Poche (10) présentant une enveloppe externe souple (12) comprenant un col (16) suivi par une partie évasée (18), le col ayant une extrémité libre (16a) munie d'une ouverture (17), la poche étant susceptible d'adopter une configuration aplatie dans laquelle l'ouverture est refermée, cependant que le col est susceptible d'adopter une configuration sensiblement tubulaire dans laquelle l'ouverture est ouverte, la poche étant, en outre, munie d'un élément de renfort (22; 122) disposé au moins au voisinage de l'extrémité libre du col,
caractérisée en ce que l'élément de renfort est constitué par un manchon relativement rigide et flexible disposé à l'intérieur de la poche (10), ce manchon s'étendant dans le col (16) à partir d'une première extrémité (22a) située au voisinage de l'ouverture (17), jusqu'à une deuxième extrémité (22b) située du côté de la partie évasée (18), en ce que le manchon présente, au voisinage de la base (16b) du col (16) raccordée à la partie évasée (18), une découpe (24) de part et d'autre de laquelle sont délimitées une première portion (23a) située du coté de la première extrémité (22a) du manchon et une deuxième portion (23b) située du côté de la deuxième extrémité (22b) dudit manchon, la deuxième portion étant munie d'au moins deux pattes (26) sensiblement transversales qui se font face et qui ont des extrémités latérales (26a) libres, et en ce que, dans la configuration aplatie de la poche, le manchon (22; 122) est lui-même aplati et les deux pattes (26) se trouvent l'une contre l'autre, tandis que, pour amener le col (16) dans sa configuration sensiblement tubulaire et ouvrir l'ouverture, le manchon est susceptible d'être déformé élastiquement de telle sorte que la première portion (23a) adopte une configuration sensiblement tubulaire, cependant que les extrémités latérales (26a) des pattes (26) sont écartées l'une de l'autre.

2. Poche selon la revendication 1, caractérisée en ce que, la première portion du manchon présentant un axe de symétrie longitudinal (D), la découpe (24) ménageant les deux pattes (26) s'étend au moins sensiblement jusqu'à cet axe de symétrie (D) à partir d'un bord longitudinal (22c) que présente ce manchon (22; 122) dans sa configuration aplatie.

3. Poche selon la revendication 1 ou 2, caractérisée en ce que le manchon (22; 122) comporte deux plaquettes (25a, 25b) analogues fixées à l'intérieur de la poche, respectivement sur deux faces internes en regard de la poche, chaque plaquette présentant une encoche sensiblement transversale (24a, 24b), de part et d'autre de laquelle s'étendent une première et une deuxième portion correspondant respectivement aux première et deuxième portions du manchon, chaque plaquette (25a, 25b) présentant deux bords longitudinaux respectivement situés au voisinage d'un premier et d'un deuxième bord longitudinal que présente le col lorsque la poche est dans sa configuration aplatie.

4. Poche selon l'une quelconque des revendications 1 à 3, caractérisée en ce que les extrémités latérales (26a) des pattes (26) sont arrondies.

5. Poche selon l'une quelconque des revendications 1 à 4, caractérisée en ce que les bords (26e) des pattes (26) qui sont tournés vers l'ouverture (17) sont sensiblement parallèles à la portion évasée (18 ; 12c) de l'enveloppe externe (12) de la poche (10).

6. Poche selon l'une quelconque des revendications 1 à 5, caractérisée en ce que le manchon (22 ; 122) comporte un prolongement (27 ; 127) s'étendant vers l'extérieur au-delà de l'ouverture (17), ce prolongement étant muni de moyens (27a, 27b ; 127a, 127b) pour faciliter le passage de la configuration aplatie à la configuration tubulaire.

7. Poche selon la revendication 6, caractérisée en ce que le prolongement (127) présente deux surfaces de préhension décalées (127a, 127b).
